Europäisches Patentamt

European Patent Office

Office européen des brevets

(11) Veröffentlichungsnummer: **0 048 323**
**B1**

(12) # EUROPÄISCHE PATENTSCHRIFT

(45) Veröffentlichungstag der Patentschrift: **24.04.85**

(21) Anmeldenummer: **81105755.3**

(22) Anmeldetag: **21.07.81**

(51) Int. Cl.⁴: **A 61 L 15/01, A 61 K 37/12, A 61 K 31/78**

(54) **Wundbehandlungsmittel in Pulverform und Verfahren zu seiner Herstellung.**

(30) Priorität: **24.09.80 DE 3036033**

(43) Veröffentlichungstag der Anmeldung:
**31.03.82 Patentblatt 82/13**

(45) Bekanntmachung des Hinweises auf die
Patenterteilung:
**24.04.85 Patentblatt 85/17**

(84) Benannte Vertragsstaaten:
**AT BE CH DE FR GB IT LI NL SE**

(56) Entgegenhaltungen:
**DE-A-2 849 570**
**FR-A-2 215 230**
**FR-A-2 302 752**
**US-A-3 963 685**

(73) Patentinhaber: **Max-Planck-Gesellschaft zur
Förderung der Wissenschaften e.V.
Bunsenstrasse 10
D-3400 Göttingen (DE)**

(72) Erfinder: **Fischer, Herbert, Prof., Dr.
Höllentalstrasse 20
D-7815 Burg (DE)**
Erfinder: **Kickhöfen, Botho, Dr.
Wildtalstrasse 56
D-7800 Freiburg (DE)**
Erfinder: **Vaubel, Ekkehard, Prof., Dr.
Klinikum Steglitz der Freien Universität Berlin
Hindenburgdamm 30 D-1000 Berlin 45 (DE)**

(74) Vertreter: **Weickmann, Heinrich, Dipl.-Ing. et al
Patentanwälte Dipl.-Ing. H.Weickmann Dipl.-
Phys.Dr. K.Fincke Dipl.-Ing. F.A.Weickmann
Dipl.-Chem. B. Huber Dr.-Ing. H. Liska Dipl.-
Phys.Dr. J. Prechtel Postfach 860820
D-8000 München 86 (DE)**

Die Akte enthält technische Angaben, die nach
dem Eingang der Anmeldung eingereicht
wurden und die nicht in dieser Patentschrift
enthalten sind.

**Beschreibung**

Wundbehandlungsmittel in Form von quellfähigen Trockenpulvern aus organischen Polymeren sind bereits bekannt. Ein derartiges bekanntes Mittel besteht aus trockenen hydrophilen Partikeln, die aus einem dreidimensionalen Netz von Dextranmolekülen bestehen und auf nässende Wunden gebracht das Wundexsudat absorbieren und zu einer gelartigen Schicht aufquellen. Hierdurch soll auch eine Schorfbildung verhindert werden. Derartige Wundpulver saugen durch Kapillarkräfte zwischen den Partikeln Bakterien an. Dies führt zwar zu einem Absaugen von Bakterien aus dem Wundgrund, ermöglicht aber andererseits auch das Eindringen von Bakterien von außen, kann also Infektionen nicht verhindern. Außerdem ist es infolge der Teilchenform oft schwierig, das Mittel wieder völlig aus der Wunde zu entfernen, obwohl dies nötig wäre.

Es besteht daher Bedarf an einem quellfähigen grannulierten Wundbehandlungspulver, welches einerseits eine starke Saugfähigkeit aufweist—und während dieser Saugphase auch Bakterien aus dem infizierten Wundgrund entfernt—welches aber andererseits im gequollenen Zustand eine gute Adhäsion zwischen den gequollenen Teilchen zeigt. so daß es als eine mehr oder weniger zusammenhängende Masse leicht aus der Wunde entfernt werden kann und gleichzeitig das Eindringen von Bakterien von außen nach innen erschwert.

Es schien zunächst unmöglich, die beiden sich scheinbar gegenseitig ausschließenden Eigenschaften, nämlich Feinteiligkeit und hohe Saugfähigkeit einerseits und Undurchdringlichkeit für Bakterien und hohe Kohäsion andererseits in einem Material zu vereinigen.

Aus FR—A 22 15 230 ist ein Mittel zum Reinigen von Flüssigkeit abgebenden Hautoberflächen, Wunden und Schleimhäuten bekannt, welches aus trockenen Teilchen eines Wasserunlöslichen, hydrophilen Polymeren besteht. Dieses Polymer soll aus Hydroxylgruppen-haltigen Molekülen bestehen. Nach den Beispielen handelt es sich um mit Epichlorhydrin vernetztes Dextran, also um Dextranomer. Dextranomerpulver hat zwar ein gute Aufnahmefähigkeit für Flüssigkeiten, diese läßt jedoch immer noch deutlich zu wünschen übrig. Außerdem ist die Kohärenz unbefriedigend, was die Entfernung aus der Wunde erschwert.

Aus der US—A 39 63 685 sind wasserunlösliche, in organischen Lösungsmitteln lösliche Hydroxyalkylmethacrylat- oder -acrylat-Polymere bekannt, die auch in Pulverform zur Herstellung von Brandwundenbandagen geeignet sein sollen. Dieses, dem HEMA-Material nahestehende Produkt ist schon aufgrund seiner Alkohollöslichkeit nicht in der Lage, den gestellten Forderungen zu genügen.

Überraschenderweise gelang es jedoch, diese Aufgabe zu lösen und ein pulverförmiges Wundbehandlungsmittel zu schaffen, welches alle diese Eigenschaften in sich vereinigt. Die Lösung dieser Aufgabe gelingt ausgehend von der in der DE—OS 28 49 570 beschriebenen Erfindung.

Aus der deutschen Offenlegungsschrift 28 49 570 ist ein transparentes Flüssigkeitsverbandmaterial bekannt, insbesondere zur Behandlung von Wunden, bestehend aus einem hydrophilen organischen transparenten Gel in Blatt- oder Bandform, welches in Form einer getrockneten quellbaren klaren Folie vorliegt, die Puffersubstanzen, in der Wundbehandlung übliche Wirkstoffe, Nährstoffe oder/und Wuchsstoffe und gegebenenfalls ein zeilen- oder netzartig angeordnetes Verstärkungsmaterial enthalten kann.

Gemäß einer bevorzugten Ausführungsform besteht das Gel aus einem gelierfähigen Polysaccharid oder/und Protein bzw. Polypeptid und einem Polymer eines hydrophilen Acryl- oder Methacrylsäure-Derivats, welches durch Polymerisation in Gegenwart des Polysacharids oder/und Proteins bzw. Polypeptids hergestellt wurde.

Nunmehr wurde gefunden, daß auf Basis dieser trockenen quellbaren Gelfolie ein pulverförmiges Wundbehandlungsmittel geschaffen werden kann, welches überraschende und vorteilhafte Eigenschaften aufweist und neue Anwendungsmöglichkeiten eröffnet.

Das erfindungsgemäße Wundbehandlungsmittel in Pulverform auf Basis eines quellfähigen organischen Polymeren ist dadurch gekennzeichnet, daß es aus einem vernetzten hydrophilen Acryl- oder Methacrylsäure-Amid polymer besteht, welches von einem gelierfähigen Polysaccharid oder/und Protein bzw. Polypeptid durchdrungen ist und durch Polymerisation von Acryl- oder Methacrylsäure-Amids in Gegenwart des gelösten gelierfähigen Polysaccharids oder/und Proteins bzw. Polypeptids und eines Vernetzungsmittels erhältlich ist.

Die Saugfähigkeit des erfindungsgemäßen Pulvers übertrifft die der bekannten pulverförmigen Wundbehandlungsmittel und die Wasseraufnahmefähigkeit beträgt ein Mehrfaches verglichen mit diesen bekannten Pulvern. Gleichzeitig aber "kleben" die gequollenen Teilchen derart aneinander, daß sie nicht nur eine kohärente und leicht wieder aus der Wunde zu entfernende Masse bilden, sondern auch eine baktieriendichte Barriere bilden, welche Krankheitserregern den Zugang zur Wunde versperrt.

Als Polysaccharid wird Agarose bevorzugt, als Protein wird Gelatine bevorzugt.

Hinsichtlich der Zusammensetzung können Gel und Polysaccharid bzw. Protein oder Polypeptid in weiten Grenzen schwanken. Bevorzugt wird jedoch eine Zusammensetzung aus 50 bis 90 Gew.-% polymerisiertem Acryl- oder Methacrylsäure-Amid und 50 bis 10 Gew.-% Polysaccharid oder/und Protein. In dieser bevorzugten Zusammensetzung weist das erfindungsgemäße Wundbehandlungsmittel eine Wasseraufnahme von mindestens 20 ml/g auf, bestimmt durch zweiminütiges Quellenlassen in salzfreiem, zweckmäßig destilliertem Wasser.

In der besonders bevorzugten Zusammensetzung besteht das erfindungsgemäße Wundbehandlungsmittel aus 50 bis 70 Gew.-% vernetztem Polyacrylamid und 50 bis 30 Gew.-% Gelatine. Bei dieser Zusammensetzung läßt sich eine Wasseraufnahme bis zu 40 ml/g unter den oben genannten Bedingungen erzielen.

Demgegenüber beträgt die Wasseraufnahme unter gleichen Bedingungen bei bekannten handelsüblichen Wundbehandlungspulvern auf Basis organischer Polymerer etwa 5 ml/g.

Das Vernetzungsmittel wird in solchen Mengen zugesetzt, daß die angestrebte Quellfähigkeit erhalten wird. Im allgemeinen werden sehr gute Quellfähigkeiten bei Verwendung von etwa 0,5 bis etwa 5 Mol-% Vernetzungsmittel, bezogen auf eingesetztes Monomeres, erhalten. Besonders günstige Ergebnisse erzielt man bei 1 bis 2 Mol-%. Bei Verwendung von Acrylamid als Monomer und Methylen-bisacrylamid als Vernetzungsmittel bedeutet dies pro Gramm Acrylamid etwa 20 bis 40 mg Methylen-bisacrylamid.

Das erfindungsgemäße Wundbehandlungspulver entwickelt in gequollenem Zustand eine so große Adhäsionskraft, daß es in den meisten Fällen aus der Wunde bereits durch Ausspülen vollständig entfernt werden kann. In gequollenem Zustand ist das Gel ausreichend transparent, um die Farbe des Wundgrunds durch das Gel hindurch beurteilen zu können.

Die Herstellung des erfindungsgemäßen Wundbehandlungsmittels erfolgt wie in der deutschen Offenlegungsschrift (DE—OS) 28 49 570 beschrieben, durch Trocknung eines Gels bestimmter Zusammensetzung und anschließendes Zerkleinern durch Mahlen bzw. Zerreiben. Um eine vollständige und rasche Trocknung zu erhalten, wird das Gel zweckmäßigerweise in zerkleinerter Form eingesetzt. Hierzu eignet sich sowohl die Folienform als auch eine Granulierung des Gels. Mit dieser zerkleinerten Form läßt sich das Gel auch rascher von niedermolekularen Bestandteilen vor dem Trocknen freiwaschen.

Die Trocknung selbst kann bei beliebigen Temperaturen zwischen Zimmertemperatur und etwa 120°C erfolgen. Oberhalb 90°C führt die Trocknung jedoch zu erheblicher Abnahme der Flüssigkeitsaufnahmefähigkeit. Besonders gute Ergebnisse wurden zwischen 40 und 80°C erhalten. Für die Trocknung selbst gelten im übrigen die Ausführungen der DE—OS 28 49 570 entsprechend.

Die Teilchengröße des erfindungsgemäßen Pulvers beeinflußt die Quellungseigenschaften. Bei abnehmender Korngröße steigt die Flüssigkeitsaufnahmegeschwindigkeit und, in geringerem Maße, auch die Menge der aufnehmbaren Flüssigkeit. Im allgemeinen wird eine Korngröße bevorzugt, bei der die Quellung im wesentlichen zwischen etwa 0,5 und etwa 5 Minuten abgeschlossen ist, was normalerweise für Korngrößen zwischen 0,05 mm und 0,5 mm zutrifft. Durch feineres oder gröberes Korn kann die Quellungsgeschwindigkeit nach Wunsch aber auch außerhalb dieses Bereichs eingestellt und dabei vergrößert oder verkleinert werden. Für abgewogene Eigenschaften bezüglich Quellungsgeschwindigkeit, Transparenz, Kohäsion und Bakteriendichte werden Mischungen verschiedener Korngrößen im angegebenen Bereich besonders bevorzugt.

Das erfindungsgemäße Wundbehandlungspulver kann als solches auf die Wunde gebracht werden. Alternativ ist es auch möglich, das Pulver zuerst mit einer begrenzten Flüssigkeitsmenge in eine pastenartige Konsistenz zu überführen und die so erhaltene Gelpaste aufzubringen. Für die Aufbringung der Paste eignen sich Spritzen sehr gut. Die Applikation mit einer solchen, Gelpaste enthaltenden Spritze ist in der Klinik sehr einfach. Das Gel wird auf und in die Wunde gespritzt und dort so lange belassen, wie dies zweckmäßig erscheint. Hierzu können auch vergefüllte sterile Fertigspritzen verwendet werden. Als Flüssigkeiten kommen die in der Wundbehandlung üblichen, vor allem physiologische Lösungen, wasserhaltige Mischungen, z.B. mit ein oder mehrwertigen Alkoholen, wie Glycerin oder organische Flüssigkeiten in Betracht, welche die in der DE—OS 28 49 570 aufgeführten Substanzen wie Arzneimittel, Nährstoffe, Desinfektionsmittel, Wuchsstoffe, Salze, Puffersubstanzen und dergleichen bei der Wundbehandlung übliche Mittel gelöst enthalten können.

Das erfindungsgemäße Pulver eignet sich auch dazu, die von der Wunde sezernierten niedermolekularen Substanzen qualitativ und quantitativ zu bestimmen. Hierzu eluiert man das aufgenommene Material nach den Methoden der Molekularsieb-Technologie, beispielsweise durch Auswaschen mit einem Salzgradienten und analysiert das eluierte Material. Um eine Verunreinigung durch ungelöste Partikel aus der Wunde zu verhindern, kann es zweckmäßig sein, das Pulver in eine semipermeable Membran, die nur für gelöste Substanzen bzw. niedrigere Molekulargewichte durchlässig ist, einzuhüllen. Je nach dem gewünschten Ausschlußgrad lassen sich hierzu üblich Dialysefolien oder Ultrafiltrationsfolien verwenden. Auch bakteriendichte Gewebe kommen in Betracht. Derartige pulverhaltige Säckchen werden als solche auf die Wunde aufgelegt und nach Aufsaugen des Sekrets entnommen, geöffnet und das Pulver in der oben beschriebenen Weise untersucht.

Die folgenden Beispiele erläutern die Erfindung weiter.

Beispiel 1
Herstellung des Trockengelpulvers
Wie im Beispiel 2 der DE—OS 27 25 261 beschrieben, wird ausgehend von 3,2 g Acrylamid, 82 mg Bisacrylamid und 2 g Agar Agar oder Agarose eine Gelplatte von einer Dicke von 3 mm hergestellt. Nach Auswaschen wird 24 Stunden bei 50°C im Trockenschrank getrocknet. Dann wird die so erhaltene spröde, getrocknete Folie in einer Kugelmühle auf eine mittlere Teilchengröße von unter 0,2 mm vermahlen. Das so erhaltene Produkt weist in destilliertem Wasser bei 2-minütiger Quellzeit eine Wasseraufnahme von 40 ml/g auf.

Die Trocknung der Gelplatte wurd wiederholt. Die Trocknungsdauer betrug 30 Minuten, die Temperatur 100°C. Nach 2 Minuten betrug die Wasseraufnahme 22 ml/g, nach 10 minuten 28 ml/g.

Mit 9 %-iger NaCl-Lösung betrug die Wasseraufnahme nach 2 Minuten für das bei 50°C getrocknete Pulver 22 ml/g.

Die Bestimmung der Wasseraufnahme erfolgte, indem in einem 25 ml-Becherglas 10 ml destilliertes Wasser vorgelegt und gewogen und die 100 mg Trockengelpulver in einem Nylonsiebgefäß in das Wasser 2 Minuten eingetaucht wurden. Dann wurde das Nylonsiebgefäß entnommen und die Gewichtsdifferenz des Becherglases gegenüber dem Gewicht vor dem Eintauchen des Pulvers bestimmt.

Beispiel 2

Wie im Beispiel 1 der DE—OS 27 25 261 beschrieben, wird ausgehend von 5 g Acrylamid, 5 g Gelatine und 130 mg N,N'-Methylen-bisacrylamid eine Gelplatte mit einer Dicke von 3 mm hergestellt. Nach Auswaschen der Gelplatte wird 60 Stunden bei 70°C im Trockenschrank getrocknet. Die so erhaltene getrocknete Folie wurde wie im Beispiel 1 beschrieben, vermahlen. Die Produkteigenschaften entsprechen denen des in Beispiel 1 beschriebenen Pulvers.

Beispiel 3

In Nährbouillon gequollenes Gel nach Beispiel 1 wurde in einem Reagenzglas mit Serratia mercescens beimpft. Als Vergleich wurde die gleiche Menge Bakterien in ein Reagenzglas mit Nährbouillon alleine eingebracht. nach 2 Tagen Bebrütung ist die Nährbouillon ohne Gel undurchsichtig, rötlich, dicht mit Bakterien bewachsen, das Reagenzglas mit Gel zeigt Bakterienwuchs nur in der obersten Schicht. Dies zeigt, daß das erfindungsgemäße Pulvergel, wenn gequollen, die Penetration von Bakterien stark erschwert oder unmöglich macht.

Beispiel 4

Ein wie in Beispiel 1 beschrieben hergestelltes erfindungsgemäßes Trockengelpulver wurde durch Siebe klassiert. Dabei wurden vier Fraktionen erhalten. Fraktion 1 bestand aus dem Anteil, der ein Sieb von 576 Maschen/cm²—etwa 0,25 mm lichte Maschenweite, nicht passierte. Die Fraktion 2 bestand aus Teilchen, die das 576-Maschensieb passierten, auf einem 900 Maschen/cm²—Sieb=etwa 0,20 mm lichte Maschenweite, zurückgehalten wurden. Fraktion 3 passierte das 900-Maschensieb und wurde durch ein 1600-Maschensieb, entsprechend etwa 0,15 mm lichte Maschenweite, zurückgehalten.

Fraktion 4 bestand aus den Teilchen, die das 1600-Maschensieb passierten.

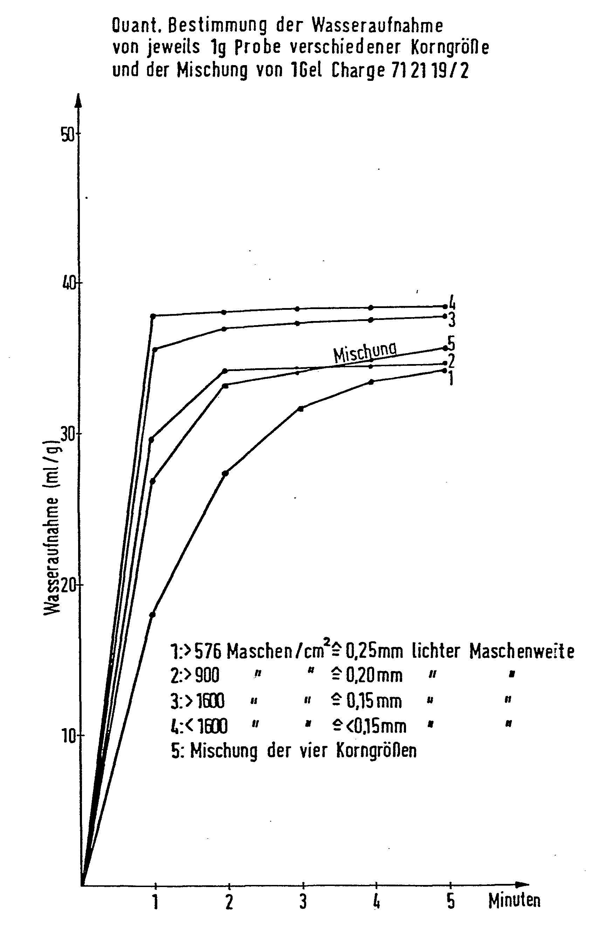

Für jede dieser vier Fraktionen und für die Ausgangsmischung wurde die Wasseraufnahme für jeweils 1 g Pulver in Abhängigkeit von der Zeit bestimmt. Die Ergebnisse sind in der Figur der beigefügten Zeichnung zusammengefaßt. Sie zeigt in graphischer Darstellung die Wasseraufnahme in ml/g, gegen Minuten aufgetragen. Die Kurven 1 bis 4 entsprechen der Wasseraufnahme der Fraktionen 1 bis 4. Kurve 5 zeigt die Wasseraufnahme der nichtfraktionierten Mischung. Letztere enthielt 68,1 Gew.-% Fraktion 1, 9,4 Gew.-% Fraktion 2, 11,4 Gew.-% Fraktion 3 und 12,1 Gew.-% Fraktion 4. Wie aus den Kurven hervorgeht, war die Wasseraufnahmegeschwindigkeit bei Fraktion 4 in der Anfangsphase etwa doppelt so groß als bei Fraktion 1, während die Gesamtwasseraufnahme nach 5 Minuten 38,3 bzw. 34,3 g betrug.

**Patentansprüche**

1. Wundbehandlungsmittel in Pulverform auf Basis eines quellfähigen organischen Polymeren, dadurch gekennzeichnet, daß es aus einem vernetzten hydrophilen Acryl- oder Methacrylsäureamid-Polymer besteht, welches von einem gelierfähigen Polysaccharid oder/und Protein bzw. Polypeptid durchdrungen ist und durch Polymerisation von Acryl- oder Methacrylsäure-Amid in Gegenwart des gelösten gelierfähigen Polysaccharids oder/und Proteins bzw. Polypeptids und eines Vernetzungsmittels erhältlich ist.

2. Wundbehandlungsmittel nach Anspruch 1, dadurch gekennzeichnet, daß das Polysaccharid Agarose ist.

3. Wundbehandlungsmittel nach Anspruch 1 oder 2 dadurch gekennzeichnet, daß das Protein Gelatine ist.

4. Wundbehandlungsmittel nach einem der vorhergehenden Ansprüche, dadurch gekennzeichnet, daß das Gel aus 50 bis 90 Gew.-% polymerisiertem Acryl- oder Methacrylsäure-Amid und 50 bis 10 Gew.-% Polysaccharid oder/und Protein besteht.

5. Wundbehandlungsmittel nach einem der vorhergehenden Ansprüche, gekennzeichnet durch eine Wasseraufnahme von mindestens 20 ml/g bei zwei minütigem Quellen in Salzfreiem Wasser.

6. Wundbehandlungsmittel nach einem der vorhergehenden Ansprüche, dadurch gekennzeichnet, daß es portionsweise von einer semipermeablen Membran umhüllt ist.

7. Wundbehandlungsmittel nach einem der vorhergehenden Ansprüche, dadurch gekennzeichnet, daß es eine Teilchengröße von 0,50 mm bis 0,05 mm aufweist.

8. Verfahren zur Herstellung des Wundbehandlungsmittels nach einem der vorhergehenden Ansprüche, dadurch gekennzeichnet, daß man Acryl- oder Methacrylsäure-Amid in einer waßrigen Lösung eines gelierfähigen Polysaccharids oder/und Proteins bzw. Polypeptids in Gegenwart eines Vernetzungsmittels und üblicher Polymerisationsinitiatoren unter Bildung eines transparenten Gels polymerisiert, das erhaltene Gel trocknet und pulverisiert.

9. Verfahren nach Anspruch 8, dadurch gekennzeichnet, daß man als Polysaccharid Agarose verwendet.

10. Verfahren nach Anspruch 8 oder 9, dadurch gekennzeichnet, daß man als Protein Gelatine verwendet.

11. Verfahren nach einem der Ansprüche 8 bis 10, dadurch gekennzeichnet, daß man das Gel bei einer Temperatur zwischen 30 und 90°C trocknet.

12. Verfahren nach Anspruch 11, dadurch gekennzeichnet, daB man bei einer Temperatur von 40 bis 80°C trocknet.

13. Verfahren nach einem der Ansprüche 8 bis 12, dadurch gekennzeichnet, daß man das Gel bis auf einen Restwassergehalt von weniger als 10 Gew.-% trocknet.

## Revendications

1. Produit sous forme de poudre pour le traitement des blessures à base d'un polymère organique apte à gonfler, caractérisé en ce qu'il est constitué d'un polymère d'acrylamide ou de méthacrylamide hydrophile réticulé, lequel est pénétré par un polysaccharide ou/et une protéine ou un polypeptide gélifiable et peut être obtenu par polymérisation d'acrylamide ou de méthacrylamide en présence du polysaccharide ou/et de la protéine ou du polypeptide gélifiable dissous et d'un agent de réticulation.

2. Produit pour le traitement de blessures selon la revendication 1, caractérisé en ce que le polysaccharide est de l'agarose.

3. Produit pour le traitement de blessures selon la revendication 1 ou 2, caractérisé en ce que la protéine est de la gélatine.

4. Produit pour le traitement de blessures selon l'une des revendications précédentes, caractérisé en ce que le gel est constitué de 50 à 90% en poids d'acrylamide ou de méthacrylamide polymérisé et de 50 à 10% en poids de polysaccharide ou/et de protéine.

5. Produit pour le traitement de blessures selon l'une des revendications précédentes, caractérisé par une absorption d'eau d'au moins 20 ml/g pour un gonflement de 2 minutes dans de l'eau ne contenant pas de sel.

6. Produit pour le traitement de blessures selon l'une des revendications précédentes, caractérisé en ce qu'il est enrobé par portions d'une membrane semi-perméable.

7. Produit pour le traitement de blessures selon l'une des revendications précédentes, caractérisé en ce qu'il présente une taille de particules de 0,50 mm à 0,05 mm.

8. Procédé pour la préparation du produit pour le traitement de blessures selon l'une des revendications précédentes, caractérisé en ce que l'on polymérise de l'acrylamide, ou du méthacrylamide dans une solution aqueuse d'un polysaccharide ou/et d'une protéine ou d'un polypeptide apte à gélifier en présence d'un agent de réticulation et d'initiateurs de polymérisation usuels avec formation d'un gel transparent, sèche le gel obtenu et le pulvérise.

9. Procédé selon la revendication 8, caractérisé en ce que l'on utilise de l'agarose en tant que polysaccharide.

10. Procédé selon la revendication 8 ou 9, caractérisé en ce que l'on utilise de la gélatine en tant que protéine.

11. Procédé selon l'une des revendications 8 à 10, caractérisé en ce que l'on sèche le gel à une température entre 30 et 90°C.

12. Procédé selon la revendication 11, caractérisé en ce que l'on sèche à une température de 40 à 80°C.

13. Procédé selon l'une des revendications 8 à 12, caractérisé en ce que l'on sèche le gel jusqu'à une teneur résiduelle en eau inférieure à 10% en poids.

## Claims

1. Wound treating agent in powder form based on a swellable organic polymer, characterised in that it consists of a cross-linked hydrophilic acrylic or methacrylic acid amide polymer which is permeated by a gellable polysaccharide and/or protein or polypeptide and is obtainable by polymerisation of acrylic or methacrylic acid amide in the presence of a dissolved, gellable polysaccharide and/or protein or polypeptide and of a cross-linking agent.

2. Wound treating agent according to claim 1, characterised in that the polysaccharide is agarose.

3. Wound treating agent according to claim 1 or 2, characterised in that the protein is gelatine.

4. Wound treating agent according to one of the preceding claims, characterised in that the gel consists of 50 to 90 wt.% of polymerised acrylic or methacrylic acid amide and 50 to 10 wt.% of polysaccharide and/or protein.

5. Wound treating agent according to one of the preceding claims, characterised by a water uptake of at least 20 ml./g. in the case of two minutes swelling in salt-free water.

6. Wound treating agent according to one of the preceding claims, characterised in that it is enveloped portionwise in a semi-permeable membrane.

7. Wound treating agent according to one of the preceding claims, characterised in that it has a particle size of 0.50 mm. to 0.05 mm.

8. Process for the production of the wound treating agent according to one of the preceding claims, characterised in that one polymerises acrylic or methacrylic acid amide in an aqueous solution of a gellable polysaccharide and/or protein or polypeptide in the presence of a cross-linking agent and of conventional polymerisation initiators with formation of a transparent gel, dries the gel obtained and pulverises.

9. Process according to claim 8, characterised in that one uses agarose as the polysaccharide.

10. Process according to claim 8 or 9, characterised in that one uses gelatine as the protein.

11. Process according to one of claims 8 to 10, characterised in that one dries the gel at a temperature between 30 and 90°C.

12. Process according to claim 11, characterised in that one dries at a temperture of 40 to 80°C.

13. Process according to one of claims 8 to 12, characterised in that one dries the gel to a residual water content of less than 10 wt.%.

Quant. Bestimmung der Wasseraufnahme
von jeweils 1g Probe verschiedener Korngröße
und der Mischung von 1Gel Charge 71 21 19/2

1:> 576 Maschen/cm$^2$ ≙ 0,25mm lichter Maschenweite
2:> 900  "  " ≙ 0,20mm  "  "
3:> 1600  "  " ≙ 0,15mm  "  "
4:< 1600  "  " ≙ <0,15mm  "  "
5: Mischung der vier Korngrößen